# EUROPÄISCHE PATENTANMELDUNG

(11) **EP 2 329 791 A2**
(43) Veröffentlichungstag der Anmeldung: **08.06.2011**
(21) Anmeldenummer: 10159504.9
(22) Anmeldetag: 09.04.2010
(51) Int. Cl.: A61C 5/06

(54) **Applikationsvorrichtung**

(30) Priorität: 04.12.2009 EP 09178109
(71) Anmelder: Ivoclar Vivadent AG, 9494 Schaan (LI)
(72) Erfinder: Müller, Frank, 6800 Feldkirch (AT); Gatehr, Klaus, 6824 Schlins (AT); Holaschke, Sebastian, 6900 Bregenz (AT); Suffel, Ralf, 9469 Haag (CH)
(74) Vertreter: Splanemann

(57) **Zusammenfassung**

Die Erfindung betrifft eine Applikationsvorrichtung, mit einem zylindrischen Grundkörper zur Bevorratung eines auspressbaren Dentalmaterials, mit einem in dem Grundkörper angeordneten, gegenüber dem Grundkörper axial versetzbaren Kolben und einer der Versetzung des Kolbens in Auspressrichtung dienenden Kolbenstange, die teilweise in den Kolben ragt und gegenüber diesem axial versetzbar ist, wobei der Kolben an seinem dem Dentalmaterial zugewandten, vorderen Endbereich mit einer in Auspressrichtung vorspannbaren, elastischen Rückstelleinrichtung versehen ist, die nach dem Ende einer jeden axialen Bewegung des Kolbens in Auspressrichtung und Aufheben des auf die Kolbenstange hierzu ausgeübten Drucks wieder ihre entspannte Stellung einnimmt und dabei einen ein Nachtropfen des Dentalmaterials aus der Applikationsvorrichtung verhindernden Unterdruck im Innern des Grundkörpers erzeugt, wobei die elastischen Rückstelleinrichtung die Kolbenstange entgegen der Auspressrichtung drückt. Die Vorspannung der Rückstelleinrichtung ist bei Betätigung der Kolbenstange (12) in Auspressrichtung durch einen in Auspressrichtung weisenden, mit der Kolbenstange (12) zusammenwirkenden, insbesondere von der Rückstelleinrichtung räumlich beabstandeten, Anschlag (18) begrenzt.

## Beschreibung

Die Erfindung betrifft eine Applikationsvorrichtung, gemäß dem Oberbegriff von Anspruch 1, die insbesondere zur Bereitstellung von Dentalmaterial in wählbaren Mengen geeignet ist.

Eine derartige Applikationsvorrichtung ist aus der DE 43 32 308 C1 bekannt. Bei dieser Lösung ist es vorgesehen, einen Stopfen vorzusehen, der eine elastische Membranfläche aufweist. Ein Drehkolben drückt die Membranfläche und mit ihr zusammen den Stopfen in Richtung einer Auslassdüse, wodurch das auszudrückende Material unter Druck gesetzt wird. Diese Drucksituation wird für die Lagerung als ungünstig empfunden, und mit der genannten Lösung soll daher nach Abschluss des Auspressvorgangs der Drehkolben so gedreht werden, dass das Material entlastet wird. Derartige Drehkolben werden als erforderlich angesehen, wenn es darum geht Viskosedentalmaterialien ohne spezielle Auspressvorrichtung, in die die Kartusche eingesetzt ist, auszudrücken. Ein Zurückdrehen wird in der Praxis jedoch regelmäßig nicht vorgenommen, oder es erfolgt sogar ein Drehen des Drehkolbens in die Ausdrückrichtung, wenn der Betätiger nicht regelmäßig die betreffende Spritze anwendet.

Bei viskosen, mittelviskosen und geringviskosen Dentalmaterialen besteht das Problem, dass aufgrund einer gewissen Materialelastizität, aber auch durch ein "Nachfließen", oder aber auch nicht vollständiges Abstreifen Dentalmaterial aus der aus der Spritze ragt, das leicht verunreinigt wird. Zwar lässt sich dieses Material bei präziser Handhabung abstreifen. Dies geschieht jedoch in der Praxis nicht in allen Fällen, so dass es nicht verwunderlich ist, dass sich auch insofern die genannte Lösung nicht durchgesetzt hat.

Ferner sind verschiedene Versuche unternommen worden, diese Nachteile der bekannten Spritzen zu beseitigen. Es ist beispielsweise vorgeschlagen worden, einen speziellen Verformungskörper in einer Spritze vorzusehen, der auf Scherung beansprucht wird und ein Rückstellmoment ausübt. Das Nachtropfen wird jedoch bei diesem Körper häufig nicht verhindert, da die Scherverformung nur in ganz geringem Maße möglich ist, so dass bei einer regelrechten Tropfenbildung dennoch gerade auch bei weniger viskosen Materialien ein Nachtropfen erfolgen kann.

Bei dieser Lösung ist es in einer alternativen Ausgestaltung vorgeschlagen worden, durch Erhöhung der Reibung zwischen einem Membranelement und der Wand der Spritze eine schleppende Führung der Membran zu realisieren. Hierzu ist das Membranelement mit einem vorgegebenen Spiel an dem eigentlichen Kolben, der sich bis zur Wand der Spritze erstreckt, gelagert, um insofern eine Führung zu ermöglichen, auch wenn das Membranelement keine eigene Stabilität aufweist.

Das Membranelement weist bei dieser Lösung einen nach hinten sich erstreckenden Flansch auf, der mit einem nach innen weisenden Vorsprung gegen eine entsprechende Form der Kolbenstange arbeitet, so dass die erwünschte Spielführung realisiert ist. Durch Betätigung des Kolbens nach vorne wird das Membranelement mittig verformt und nach vorne gewölbt. Diese Verformung ist jedoch ausgesprochen nachteilig für die Anlage am Außenrand, also die Anlage zwischen Membranelement und Spritze. Hierdurch wird nämlich das Membranelement gleichsam nach innen gezogen, so dass leicht Fremdsubstanzen wie das auszudrückende Dentalmaterial in den Spalt zwischen Membranelement und Wand gelangen können und die erwünschte Dichtung zunichte machen.

Zwar mag der rückwärtige Bereich des Membranelements zunächst noch anliegen. Da er jedoch von der Kolbenstange nicht abgestützt ist, verschiebt sich der praktisch keilförmige Verlauf des aufgenommenen in dem Rand befindlichen Dentalmaterials beim Ausdrücken des Dentalmaterial nach hinten, so dass die erwünschte Dichtwirkung zunichte gemacht wird, was auch leicht zu einem Verklemmen bzw. sogar zur Zerstörung des Membranelements führen kann.

An sich ist es bekannt, einen Kolben über eine Spritze mit einer Dichtung, wie beispielsweise einer O-Ring-Dichtung abzudichten. Hier muss stets sorgfältig darauf geachtet werden, dass keine Verunreinigungen die Dichtfunktion selbst beeinträchtigen können, insbesondere, wenn mit höheren Drücken gearbeitet wird.

Das Dentalmaterial, das von den erfindungsgemäßen Applikationsvorrichtungen aufzubringen ist, kann sowohl in recht flüssiger als auch in recht viskoser Form vorliegen.

Demgegenüber liegt der Erfindung die Aufgabe zugrunde, eine Applikationsvorrichtung gemäß dem Oberbegriff von Anspruch 1 zu schaffen, die hinsichtlich der Bedienbarkeit verbessert ist, und eine breite Bandbreite der Viskosität abdeckt und auch störungsfreier arbeitet.

Diese Aufgabe wird erfindungsgemäß durch Anspruch 1 gelöst. Vorteilhafte Weiterbildungen ergeben sich aus den Unteransprüchen.

Erfindungsgemäß ist es vorgesehen, einen speziellen Kolben sicher zu führen, der hülsenförmig ausgebildet ist und an seiner Innenseite flächig abgestützt ist. Hierdurch ist sichergestellt, dass auch bei Zugwirkung der Kolbenstange auf den zentralen Bereich eines elastischen Rückstellelement des Kolbens dieses im Umfangsbereich sich nicht radial kontrahiert, sondern lediglich axial gleitet, so dass an dieser Stelle Dentalmaterial hereingelangen kann. Durch den vorgegebenen, von der Rückstelleinrichtung räumlich beabstandeten Anschlag ist eine definierte Relativbewegung zwischen Kolbenstange und Kolben gewährleistet, wobei es besonders günstig ist, dass der Anschlag und der Gegenanschlag durch vergleichsweise harte Materialien gebildet sind, die auch bei sehr viskosem Material einen sicheren Vortrieb gewährleisten, ohne dass das recht weiche elastische Rückstellelement überbeansprucht würde. Erfindungsgemäß lässt sich daher eine großflächige Abstützung des elastischen Rückstellelements durch den Kolben gewährleisten und dass eine Überanspruchung von membranartigen Elementen sicher vermeiden.

Erfindungsgemäß ist es in diesem Zusammenhang besonders günstig, dass das elastische Rückstellelement sich von der Anlage des Kolbens an dem Grundkörper der Applikationsvorrichtung, also dem Spritzenkörper, unabhängig verformen kann. Insbesondere zieht die Auswölbung der Membran durch die dort eintretende Kolbenstangenspitze das Kolbenelement nicht nach innen, so dass die dort in einer nach außen weisenden Ringnut eingelegte Dichtung eine sichere Abdichtung gegenüber dem Grundkörper ermöglicht.

Erfindungsgemäß ist in einer vorteilhaften Ausgestaltung insofern die reine Dichtfunktion von der Verformungsfunktion getrennt, was auch insofern besonders günstig ist, da die eingesetzten Materialien je optimiert werden können, ohne auf die je andere Funktion Rücksicht nehmen zu müssen.

Beispielsweise kann für einen Kolbenkörper des Kolbens ein recht hartes und formstabiles Kunststoffmaterial gewählt werden. Demgegenüber ist das elastische Rückstellelement deutlich weicher und elastisch verformbar. Das das elastische Rückstellelement kann bevorzugt als vorderer Teil des Kolbens ausgebildet sein, und der Kolbenkörper erstreckt sich bevorzugt über den vorderen Teil der Länge der Kolbenstange, beispielsweise über die vorderen 20%, so dass auch insofern auch eine sichere und reibungsarme Führung gewährleistet ist.

In vorteilhafter Ausgestaltung ist die Relativbewegung zwischen Kolbenstange und Kolben bevorzugt von außen sichtbar. Der Zahnarzt oder Zahntechniker, der die erfindungsgemäße Applikationsvorrichtung einsetzt, kann so gleichsam automatisch erkennen, dass die Kolbenstange durch Druck auf das erfindungsgemäße Betätigungselement sich in den Kolben stärker einführen lässt, so dass die Auswölbung der Membran praktisch von außen sichtbar ist. Hierzu kann entweder der Kolben in den rückwärtigen Bereich der Kolbenstange hinaus verlängert sein, oder es kann über ein schlitzförmiges Fenster im Grundkörper die Bewegung des Kolbens sichtbar gemacht werden.

Besonders günstig ist es, dass gleichsam automatisch durch Entfernen des Drucks auf die Kolbenstange sichergestellt ist, dass der Druck auf das Dentalmaterial im Innenraum der Applikationsvorrichtung aufgehoben ist, so dass der Dentaltechniker dann unbesorgt die Applikationsvorrichtung vom Applikationsort entfernen kann, ohne befürchten zu müssen, dass Dentalmaterial nachtropft.

Überraschend wird durch die erfindungsgemäße Kombination einer Membran mit signifikanten Rückstellkräften, die größer sind als die Reibungskräfte einer Kolbenstange, eine störungsfreie, einfache und dennoch nachtropfsichere Spritze oder Applikationsvorrichtung bereitgestellt: Durch die Realisierung des hülsenförmigen Kolbens lässt sich eine sichere Führung über die gesamte Länge der Applikationsvorrichtung gewährleisten, und es steht eine vergleichsweise große Führungsfläche zur Verfügung, die das Dentalmaterial nach vorne drückt und an der Stirnseite der Kolbenstange die erfindungsgemäße, freifedernde Rückstellmembran in die als Rückstellelement richtige Richtung drückt. Erfindungsgemäß ist es in diesem Zusammenhang besonders günstig, wenn der hülsenförmige Kolben zwar zum Auspressen des Dentalmaterials über die Membran von der Kolbenstange mitgeschleppt wird, jedoch gegenüber der Innenwand des Grundkörpers eine größere Reibung als die Reibung zwischen Kolbenstange und Kolben aufweist. Damit ist sichergestellt, dass beim Rückfedern der Rückstelleinrichtung die Kolbenstange bewegt wird, nachdem sie in dem Kolben leicht verschieblich ist.

Erfindungsgemäß lässt sich dieses Merkmal insbesondere durch drei symmetrisch verteilte Rippen gewährleisten, die sich mit geringer Anlagefläche, beispielsweise ballig, zur Kolbenstange hin erstrecken und insofern vom Kolben hin radial einwärts vorspringen. Es versteht sich, dass anstelle dessen auch eine kinematische Vertauschung vorgenommen werden kann, so dass Rippen von der Kolbenstange ausgehen und sich zum Kolben hin erstrecken.

Erfindungsgemäß besonders günstig ist es, wenn die Kolbenstange eine im Wesentlichen plane Stirnfläche aufweist, die das Rückstellelement, beispielsweise in dem zentralen Bereich - flächig abstützt. Diese Ausgestaltung hat den besonderen Vorteil, dass das Rückstellelement während des Vortriebs, in welchem Zustand das Rückstellelement am stärksten auf Druck belastet ist, sich flach erstrecken kann und nicht gedehnt wird. Die Erstreckung der zentralen Stirnfläche kann sich entweder über nahezu die gesamte freie Fläche - abgesehen von einem Randspalt - zum Kolben hin erstrecken oder beispielsweise etwa die Hälfte des Durchmessers des Rückstellelements betragen. Endseitig der Stirnfläche ist bevorzugt eine Fase oder noch weiter bevorzugt ein Radius vorgesehen, der eine sanfte Umlenkung des Rückstellelements dann ermöglicht, wenn der Vortrieb der Kolbenstange auf derart großen Wiederstand stößt, dass der Kolben relativ zur Stirnfläche der Kolbenstange etwas zurückgedrückt wird.

Bevorzugt ist jedenfalls, dass die freie Stirnfläche des Rückstellelements, also die dem Dentalmaterial zugewandte Fläche, sich hinterschneidungsfrei erstreckt, wobei es möglich ist, im entspannten Zustand eine konkave oder konvexe oder plane Vortriebs-Stirnfläche bereitzustellen, je nach den Anforderungen des auszudrückenden Dentalmaterials.

In vorteilhafter Ausgestaltung der erfindungsgemäßen Applikationsvorrichtung ist es vorgesehen, dass die Rückstellkraft des Rückstellelements größer als die Verformungsreibung des Dentalmaterials ist. Das Dentalmaterial wird dadurch auch bei einer starken Viskosität zurückgesaugt und es besteht die Sicherheit, dass kein Nachtropfen zu befürchten ist.

In weiterer vorteilhafter Ausgestaltung ist es vorgesehen, das elastische Rückstellelement an den zentralen Bereich mit einer größeren Wandstärke zu versehen. Die größere Wandstärke erlaubt es, den durch die Kompression entstehenden Druck gleichmäßig zu verteilen. Besonders günstig ist es in diesem Zusammenhang, wenn die Wandstärkenverdickung insofern im Querschnitt trapezförmig ist, denn hierdurch wird die Krafteinleitung noch weiter verbessert, so dass der von der Kolbenstange von deren vorderen Stirnfläche ausgeübte Druck noch besser gleichmäßig auf das Rückstellelement verteilt wird. Das Rückstellelement ist bevorzugt im Wesentlichen hauben- oder topfförmig, wobei der den zentralen Bereich umgebende Bereich mit dünnerer Wandstärke und gleicher Wandstärke wie der zylindrische Bereich besonders elastisch ist, so dass die erwünschte Elastizität ohne Weiteres sichergestellt sein kann, und insbesondere ohne dass Spannungsspitzen im Material auftreten würden, die ein Einreißen des elastischen Rückstellelements befürchten lassen würden.

Die Verbindung kann insofern kraft- und/oder formschlüssig realisiert sein und lässt sich im weiten Bereich an die Erfordernisse anpassen.

Die Kolbenstange kann im Bereich ihrer Kolbenstangenspitze eine Form aufweisen, die eine Verformung der Membran um ein recht großes Volumen zulässt, ohne die Membran punktuell stark zu beanspruchen. Die Kolbenstange kann insofern nach der Art einer Druckstange ausgebildet sein und vorne etwas konisch zulaufen. Dennoch kann sie eine im Wesentlichen flache Stirnfläche aufweisen, die gegenüber dem Konus mit einem Radius oder einer Fase abgeschlossen sein kann.

In vorteilhafter Ausgestaltung ist das Rückstellelement im entlasteten Zustand an ihrer Vorderseite plan, so dass sie an der ebenfalls planen Stirnfläche der Kolbenstange flach anliegen kann. In einer alternativen Ausgestaltung ist sie im entlastenden Zustand konkav, also zur Kolbenstange hin gewölbt. Die Kolbenstangenspitze kann bei dieser Ausführungsform ebenfalls plan sein, und es besteht die Möglichkeit, dass sich die Membran durch die "Nullposition", also die plane Position, hindurch vorwölbt, um das Dentalmaterial auszudrücken, was den Hub der Membran nahezu verdoppelt.

In vorteilhafter Ausgestaltung weist der Kolbenkörper ferner eine außen umlaufende Ringnut auf, die einen Dichtungsring aufnimmt. Die umlaufende Dichtung gewährleistet eine flüssigkeitsdichte Abdichtung, während das vergleichsweise harte Kolbenelement bei viskosen Dentalmaterialien dafür sorgt, dass diese nicht in den Spalt zwischen den Kolbenelement und Grundkörper eindringen können.

In vorteilhafter Ausgestaltung ist es vorgesehen, dass in der entspannten Stellung der Rückstelleinrichtung die Kolbenstange an einem entgegen der Auspressrichtung weisenden Anlagebereich der Rückstelleinrichtung anliegt und der Anschlag des Kolbens von einen entsprechenden Gegenanschlag der Kolbenstange entsprechend dem Weg der maximalen Vorspannung der Rückstelleinrichtung beabstandet ist.

In vorteilhafter Ausgestaltung ist es vorgesehen, dass insbesondere der Anlagebereich des Rückstellelements beim Aufbringen einer Auspresskraft von der Kolbenstange in Auspressrichtung vorspannbar ist.

In vorteilhafter Ausgestaltung ist es vorgesehen, dass die Rückstelleinrichtung in Anlagebereich eine größere Wandstärke aufweist.

In vorteilhafter Ausgestaltung ist es vorgesehen, dass die elastische Rückstelleinrichtung insbesondere mit einem verjüngten Außenumfang des Kolbens fest verbunden ist.

In vorteilhafter Ausgestaltung ist es vorgesehen, dass Kolben und Kolbenstange gewindefrei translatorisch zueinander und zu dem Grundkörper gewindefrei axial verschieblich gelagert sind und die Treibkraft des Kolbens gegenüber dem Grundkörper größer als die Widerstandskraft des elastischen Rückstellelements gegen Verformen ist.

In vorteilhafter Ausgestaltung ist es vorgesehen, dass am Außenumfang des Kolbens mindestens zwei Dichtrippen umlaufend ausgebildet sind, die gegen den Grundkörper wirken, und dass insbesondere an der Kolbenstange und/oder an dem Kolben eine umlaufende Aufnahmenut oder eine Aufnahmeschulter für die Aufnahme eines Dichtrings aus einem elastischen Material ausgebildet ist.

In vorteilhafter Ausgestaltung ist es vorgesehen, dass die Kolbenstange sich durch den Kolben hindurch bis zum Anlagebereich der Rückstelleinrichtung erstreckt und der Anschlag zwischen Kolbenstange und Kolben im Verlauf des Kolbens, bevorzugt etwa in dessen axialer Mitte, gebildet ist.

In vorteilhafter Ausgestaltung ist es vorgesehen, dass der Kolben an seinem rückwärtigen Ende mindestens eine Gleitrippe aufweist, über welche er an der Kolbenstange reibungsarm abgestützt ist.

In vorteilhafter Ausgestaltung ist es vorgesehen, dass die Applikationsvorrichtung eine Ausbringdüse aufweist, die an einer in Auspressrichtung weisenden Seite des Grundkörpers befestigt ist.

In vorteilhafter Ausgestaltung ist es vorgesehen, dass der Grundkörper bei abgenommener Ausbringdüse befüllbar ist, und der Kolben und die Kolbenstange beim Befüllen zurückgedrückt wird.

In vorteilhafter Ausgestaltung ist es vorgesehen, dass die Kolbenstange wenigstens eine umlaufende Nut aufweist, die der Aufnahme eines sich an der Innenwandung des Grundkörpers dichtend anliegenden Dichtungen dient.

Weitere Vorteile, Einzelheiten und Merkmale ergeben sich aus der nachfolgenden Beschreibung mehrerer Ausführungsbeispiele der Erfindung anhand der Zeichnungen.

Es zeigen:
- Fig. 1: zeigt eine erste Ausführungsform einer erfindungsgemäßen Applikationsvorrichtung, wobei Kolben und Kolbenstange sich in der zurückgezogenen Position befinden;
- Fig. 2: zeigt eine vergrößerte Darstellung eines Details der ersten Ausführungsform gemäß Fig. 1;
- Fig. 3: zeigt eine zweite Ausführungsform einer erfindungsgemäßen Applikationsvorrichtung, bei der Kolbenstange und Kolben zurückgezogen sind;
- Fig. 4: zeigt die Applikationsvorrichtung gemäß Fig. 3, wobei die Kolbenstange zum Ausdrücken des Dentalmaterials nach vorne gedrückt ist;

- Fig. 5: zeigt eine vergrößerte Darstellung des vorderen Endes der Kolbenstange und der umgebenden Bereiche der Applikationsvorrichtung, in der Position gemäß Fig. 3; und
- Fig. 6: zeigt eine vergrößerte Darstellung des vorderen Endes der Kolbenstange und der umgebenden Bereiche der Applikationsvorrichtung, in der Position gemäß Fig. 4.

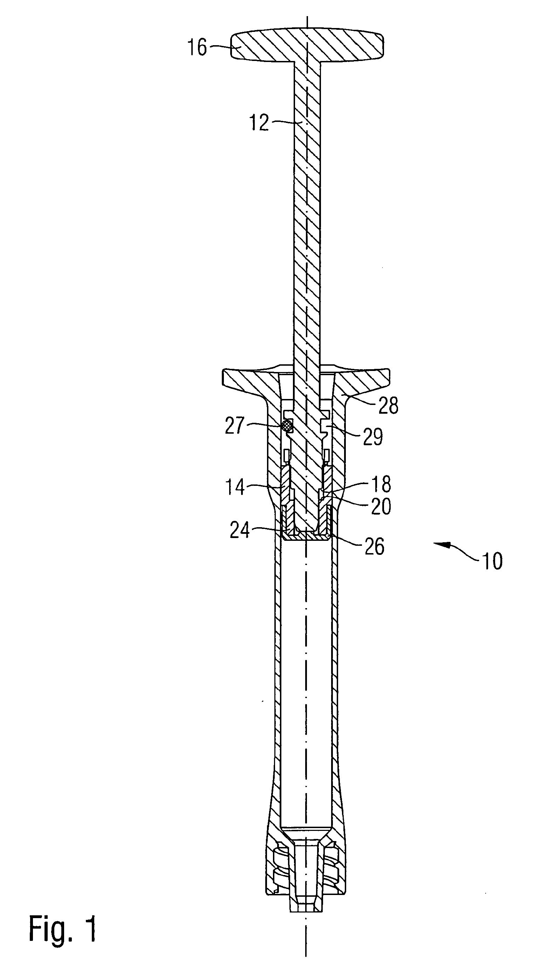

Die in Fig. 1 dargestellte Applikationsvorrichtung 10 weist eine Kolbenstange 12 und einen Kolben 14 auf. Die Kolbenstange 12 ist in dem Kolben 14 axial beweglich geführt und endet in ihrem rückwärtigen Bereich an einem Betätigungselement 16, das nach der Art eines Handgriffs eines Spritzenkolbens ausgebildet ist und mit welcher Kolbenstange 12 und der Kolben 14 nach vorne gedrückt werden können.

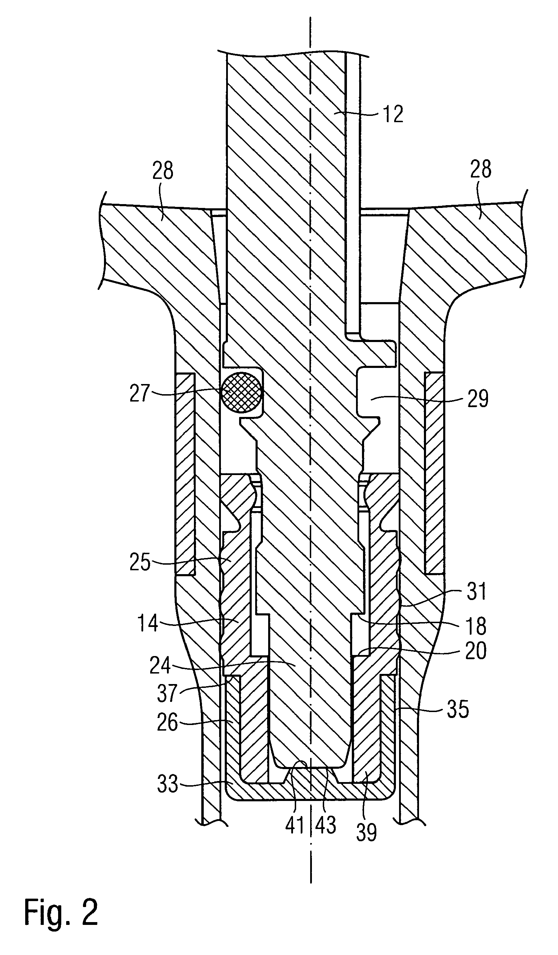

Hierzu weist die Kolbenstange 12 einen Anschlag 18 auf, der auf einen Gegenanschlag 20 des Kolbens 14 wirkt. Die Kolbenstange 12 endet an ihrem vorderen Ende in einer Kolbenstangenspitze 24. Hier endet der Kolben 14 in einem elastischen Rückstellelement 26, das fest mit einem Kolbenkörper 25 verbunden ist, an dem der Gegenanschlag 20 angeordnet ist. Das elastische Rückstellelement 26 als Teil einer Rückstelleinrichtung für die Kolbenstange 12 ist unter Bezugnahme auf Fig. 2 näher beschrieben.

Der Kolbenkörper 25 weist umlaufend Rippen 31 auf, die aus Fig. 2 ersichtlich sind und gegen einen Grundkörper 28 abdichten. Zusätzlich ist eine Ringnut 29 vorgesehen, in die bei Bedarf ein O-Ring 27 einlegbar ist.

Aus Fig. 2 ist ersichtlich, in welcher Weise sich die Kolbenstange 12 mit ihrer Kolbenstangespitze 24 in den Kolben 14 hinein erstreckt. Ferner ist auch der Aufbau des Kolben 14 im einzelnen ersichtlich. Wie bereits festgehalten, besteht der Kolben aus einem Kolbenkörper 25 und einem elastischen Rückstellelement 33. In der Montageposition an dem Kolbenkörper 25 bildet das elastische Rückstellelement 33 die elastische Rückstelleinrichtung. Hierzu ist das elastische Rückstellelement 33, das aus dem weicheren und elastischeren Material als der Kolbenkörper 25 im Übrigen besteht, einen im Wesentlichen hauben- oder topfförmigen Aufbau. Ein zylindrischer Bereich 35 endet stirnseitig an einem Absatz 37 des Kolbenkörpers 25. Dort ist eine Verbindung zwischen elastische Rückstellelement 33 und Kolbenkörper 25 vorgesehen, entweder durch Kleben oder in einer beliebiger anderen geeigneten Weise, wobei es auch möglich ist, die beiden Teile 25 und 33 einstückig herzustellen, besonders bevorzugt durch Spritzguss unter Verwendung unterschiedlicher Materialien.

Der Kolbenkörper 25 weist einen rohrförmigen Abschnitt 39 auf, der an seiner Außenseite den zylindrischen Bereich 35 des elastische Rückstellelement 33 flächig führt. Damit ist eine axiale Bewegung zwischen dem elastische Rückstellelement 33 und dem Kolbenkörper 25 bei Dehnung des elastischen Rückstellelements 33 möglich.

Das topf- oder haubenförmige elastische Rückstellelement 33 weist abgesehen von einem Anlagebereich 41 überall die gleiche Wandstärke auf. Der Anlagebereich 41 ist als Verdickung ausgebildet, die der Kolbenstangenspitze 24 zugewandt ist und an einer Stirnseite 43 an dem vorderen Endbereich, also der Kolbenstangenspitze 24 der Kolbenstange 12 anliegt.

Der Anlagebereich 41 des elastischen Rückstellelements 33 erstreckt sich in radialer Richtung über weniger als die Hälfte des Durchmessers des elastischen Rückstellelements 33, bevorzugt etwa über lediglich 1/3 seines Durchmessers. Damit ist eine besonders günstige Anlage mit geringer Reibung realisiert, die in Vorwölben des elastischen Rückstellelements 33 zum Dentalmaterial hin begünstigt. Die Vorwölbung ist durch den Anschlag 18 und den Gegenanschlag 20 an dem Kolbenkörper 25 bzw. der Kolbenstange 12 begrenzt, wobei das Vorwölbmaß durch den Abstand zwischen diesen in der Ruhestellung der Kolbenstange 12 begrenzt ist.

Bei Vorwölbung wird nun die eingeleitete Kraft über den Anlagebereich 41 besonders günstig das elastische Rückstellelement 33 eingeleitet. Hierzu hat der Anlagebereich einen im Wesentlichen trapezförmigen Querschnitt, mit dem kürzeren Trapezschenkel an der Stirnseite 43 und dem längeren Trapezschenkel zum elastischen Rückstellelement 33 im Übrigen hin. Die eingeleitete Kräfte werden so großflächig verteilt, obwohl eine vergleichsweise geringe Anlagefläche zwischen dem elastischen Rückstellelement 33 und der Kolbenstangenspitze 24 vorliegt.

Durch die Gleitfläche, die zwischen dem zylindrischen Bereich 34 und dem rohrförmigen Abschnitt 39 besteht, steht eine ausgesprochen große Dehnlänge zur Verfügung, die die Kraftverteilung der eingeleiteten Druckkraft über die Kolbenstange 12 weiter begünstigt.

Besonders günstig ist es aber, dass die Kolbenstange 12 automatisch zurückfedert und damit zugleich automatisch Dentalmaterial zurücksaugt, wenn sie entlastet wird. Hierzu ist es vorgesehen, dass die Reibung an den Rippen 31 zwischen dem Kolben 14 und dem Kolbenkörper 28 größer ist als die Federkraft, die elastische Rückstellelement 33 auf die Kolbenstangenspitze 24 ausübt, und zwar bis der Anschlag 18 dem Gegenanschlag 20 erreicht. Erst beim Erreichen dieser stabilen Anlage beginnt der Kolben 14 sich zusammen mit der Kolbenstange 12 zu bewegen, und wenn er die Zielposition erreicht hat, federt die Kolbenstange 12 automatisch zurück.

Die Relativbewegung zwischen Kolbenstange und Kolben ist bevorzugt von außen sichtbar, und zwar auch dann, wenn die Kolbenstange 12 und der Kolben 14 vollständig in die Applikationsvorrichtung eingedrückt sind. Anstelle der hier realisierten Kombination des Druckkörpers 16 mit dem Anschlag 22 lässt auch über ein Fenster in dem Grundkörper 28 die Relativposition zwischen Kolben und Kolbenstange sichtbar machen.

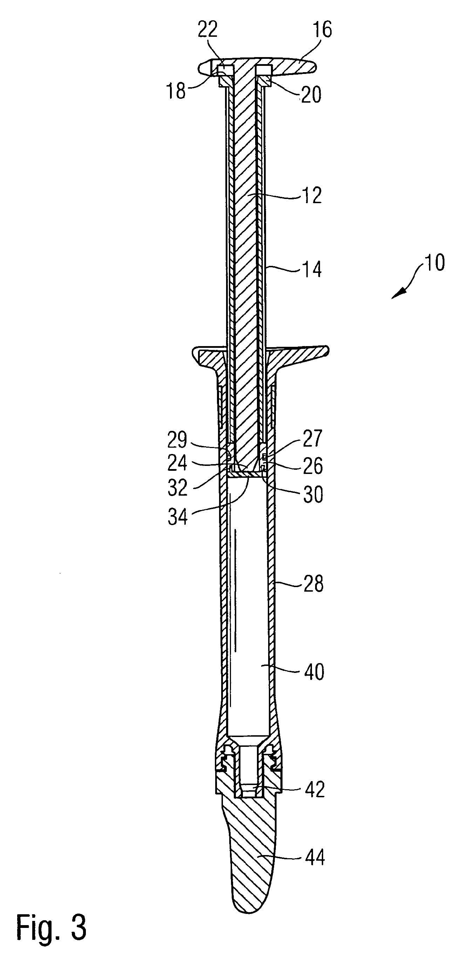

Aus Fig. 3 ist eine modifizierte Ausgestaltung einer erfindungsgemäßen Applikationsvorrichtung ersichtlich. Bei dieser Lösung erstreckt sich der Kolben 14 über im Wesentlichen die gesamte Länge der Kolbenstange 12, und der Anschlag 18 ist im rückwärtigen Bereich an einem Druckkörper 16 ausgebildet, der hier eine hierfür geeignete ringförmige Ausnehmung 22 aufweist, in die der Kolben 14 mit seinem Gegenanschlag 20 eintreten kann.

Auch bei dieser Lösung ist ein elastisches Rückstellelement 33 vorgesehen, das lediglich in einem zentralen Bereich vom Druck der Kolbenstange 12 beaufschlagt wird und sich verformt, wobei auch hier die Reibungskräfte zwischen dem Kolben 14 und dem Grundkörper 28 größer sind als die Reibungskräfte zwischen der Kolbenstange 12 und dem Kolben 14 plus der Rückstellkräfte des elastischen Rückstellelements 33.

Wenn die Kolbenstangenspitze 24 und das elastische Rückstellelement 33 zueinander in einer Relativposition sind, in der beide nach vorne hin an der gleichen Position enden, ist der Druckkörper an sich noch nicht in die Ausnehmung 22 des Betätigungselements 16 eingetreten; der Abstand, bis der Anschlag 18 erreicht ist, beträgt etwa den halben Durchmesser der Kolbenstange 12.

In dem elastischen Rückstellelement 33 ist eine Stirnnut 30 in über Stirnfläche ausgebildet, die einen Ringflansch 32 einer Membran 34 aufnimmt.

Die Membran 34 ist elastisch ausgebildet und weist eine Stärke von deutlich weniger als der Hälfte des Durchmessers der Kolbenstange 12 auf, beispielsweise 1/10 bis 1/3 und besonders bevorzugt etwa 1/5 des Durchmessers.

Die Membran 34 ist elastisch und in dem in Fig. 3 dargestellten Zustand, bei dem sich die Kolbenstangenspitze 24 und die Membran 34 plan zueinander erstrecken, liegt die Kolbenstangenspitze 24 auf der Membran 34 auf, jedoch ohne sie zu verformen.

In dem Innenraum 40 des Druckkörpers 28 ist vor der Membran 34 Dentalmaterial aufgenommen, das über eine Düse 42 aus der Applikationsvorrichtung 10 ausdrückbar ist. In dem in Fig. 3 dargestellten Zustand ist die Düse 42 in an sich bekannter Weise mit einer Kappe 44 verschlossen, um ein Austrocknen des Dentalmaterial in dem Innenraum zu verhindern.

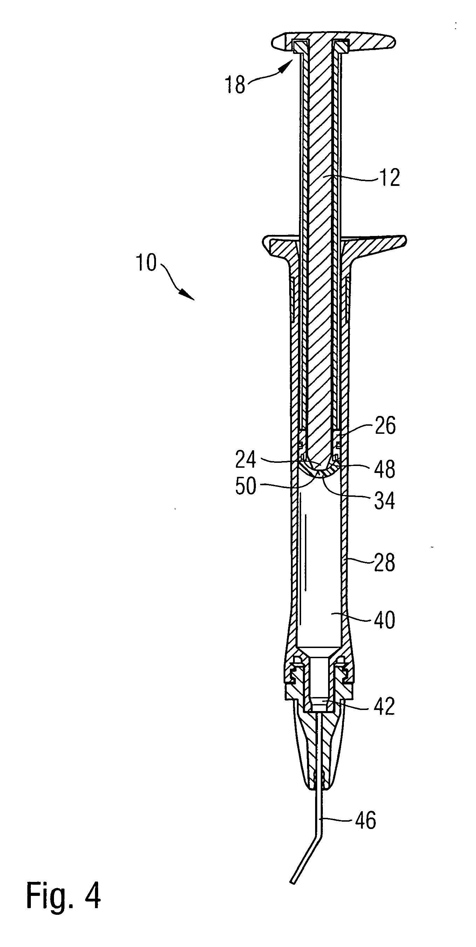

In Fig. 4 ist dargestellt, wie das Ausdrücken des Dentalmaterials aus der Applikationsvorrichtung 10 von statten geht. Für das Applizieren des Dentalmaterials wird auf die Düse 42 ein Spritzenelement 46 aufgesteckt, dessen Ausgestaltung in weiten Bereichen an die Erfordernisse anpassbar ist. Wenn beispielsweise eine Wurzelbehandlung eines Zahns vorgenommen werden soll, wird ein hierfür geeignetes Spritzenelement mit einem nadelförmigen Ende mit Hohlkanal verwendet.

Das Spritzenelement 46 hat einen vorgegebenen Strömungswiderstand, der in starkem Maße von der Viskosität des Dentalmaterials in dem Grundkörper 28 abhängt. Erfindungsgemäß lässt sich sowohl recht dünnflüssiges Dentalmaterial über ein Spritzenelement mit einer engen Durchlassöffnung als auch viskoses Dentalmaterial über die Düse 42 oder ein Spritzenelement mit einem entsprechend großen Querschnitt applizieren, oder aber sogar dünnflüssiges Dentalmaterial durch einen vergleichsweise großen Querschnitt hindurch. Die erfindungsgemäße Applikationsvorrichtung bietet insofern ein breites Anwendungsspektrum unterschiedlicher Appliziersituationen und Dentalmaterialien.

Erfindungsgemäß ist es hierzu vorgesehen, die Membran 34 durch die Kolbenstange 12 gegenüber dem elastisches Rückstellelement 33 sich vorwölben zu lassen, und zwar soweit, bis der Anschlag 18 zwischen dem Druckkörper 16 und der Kolbensstange 12 erreicht ist.

Die Membran 34 wird durch die Kolbenstangenspitze 24 nach vorne gedrückt, so dass sie sich bauchig zum Dentalmaterial hin vorwölbt. In diesem Zusammenhang ist es besonders günstig, wenn die Kolbenstangenspitze 24 einen Führungskonus 48 aufweist, der über einen kleinen Radius zu einer Stirnfläche 50 der Kolbenstangenspitze übergeht. Der Führungskonus 48 reduziert die Scherbelastung der Membran 34, die dementsprechend lediglich in ihrem zentralen Bereich von der Kolbenstange 12 mit Druck beaufschlagt wird.

Die Vorwölbung der Membran 34 wird durch die Wirkung des Anschlags 18 begrenzt. Dies bedeutet, dass die Membran 34 nie extrem gedehnt wird und vergleichsweise dickwandig ausgebildet sein kann. Das maximale Vorsprungmaß entspricht beispielsweise etwa einem zehntel bis 2/3 des Durchmessers der Kolbenstange 12, insbesondere etwa 1/3 oder 1/2 des Durchmessers der Kolbenstange 12.

Sobald die Kolbenstange 14 entlastet wird, also sobald das Betätigungselement 16 von dem Benutzer freigegeben wird, kommt die Federwirkung der Membran 34 zum Tragen. Die Membran 34 verschiebt nicht nur die Kolbenstange 12 nach rückwärts, also in Richtung des Betätigungselements 16 hin, sondern vergrößert auch den wirksamen Innenraum 40 des Grundkörpers so dass dort Unterdruck entsteht. Hierdurch wird wiederum Dentalmaterial durch das Spritzenelement 46 rückwärts gesaugt, so dass das Nachtropfen der Applikationsvorrichtung sicher verhindert wird.

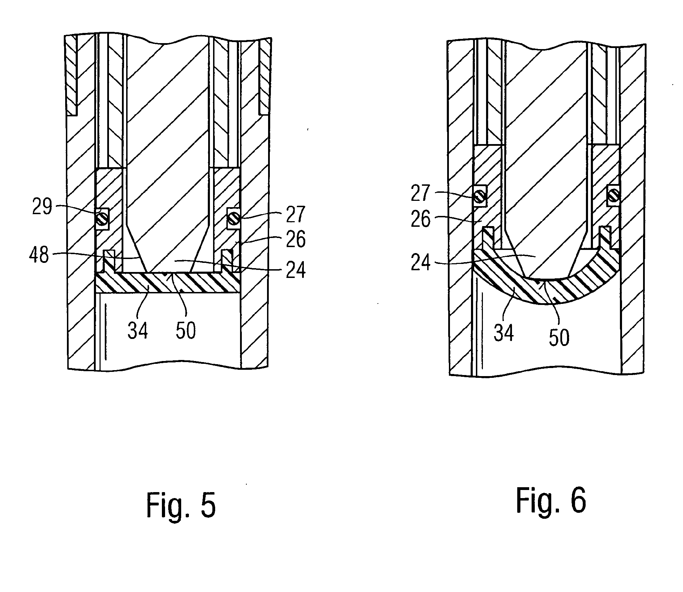

Aus Fig. 5 und 6 sind die Ausgestaltung des elastischen Rückstellelements 33 und der Kolbenstangenspitze 24 in vergrößerter Darstellung ersichtlich. Durch die flache Stirnfläche 50 an der Vorderseite der Kolbenstangenspitze 24 wird eine punktuelle Überlastung der Membran 34 verhindert; diese wird vielmehr großflächig unter Druck gesetzt und drückt das Dentalmaterial aus.

Der Konuswinkel des Führungskonus 48 beträgt in dem dargestellten Ausführungsbeispiel lediglich etwa 30 °. Es versteht sich, dass auch ein wesentlich größerer Konuswinkel realisierbar ist, beispielsweise etwa 120 °, da auch in diesem Fall sichergestellt ist, dass die Membran bauchig belastungsarm verformt wird. Auch ist es möglich, die Stirnfläche 50 mit einem Radius zu kombinieren, der bis zum Durchmesser der Kolbenstangenspitze 24 verläuft, wobei sichergestellt sein muss, dass keine Überlastung der Membran 34 erzeugt wird.

Die Anhand der Fig. 3 bis 6 erläuterten Vorteile und Einzelheiten gelten sinngemäß auch für die Ausführungsform gemäß den Fig. 1 und 2, insbesondere auch hinsichtlich der Ausgestaltung der Düse 42 und der Dehnungsbeschränkung des elastischen Rückstellelements 33, wobei es sich versteht, dass die Kraftverteilung in das elastische Rückstellelement 33 bei der Ausführungsform gemäß Fig. 1 und 2 noch verbessert ist.

## Patentansprüche

1. Applikationsvorrichtung, mit einem zylindrischen Grundkörper zur Bevorratung eines auspressbaren Dentalmaterials, mit einem in dem Grundkörper angeordneten, gegenüber dem Grundkörper axial versetzbaren Kolben und einer der Versetzung des Kolbens in Auspressrichtung dienenden Kolbenstange, die teilweise in den Kolben ragt und gegenüber diesem axial versetzbar ist, wobei der Kolben an seinem dem Dentalmaterial zugewandten, vorderen Endbereich mit einer in Auspressrichtung vorspannbaren, elastischen Rückstelleinrichtung versehen ist, die nach dem Ende einer jeden axialen Bewegung des Kolbens in Auspressrichtung und Aufheben des auf die Kolbenstange hierzu ausgeübten Drucks wieder ihre entspannte Stellung einnimmt und dabei einen ein Nachtropfen des Dentalmaterials aus der Applikationsvorrichtung verhindernden Unterdruck im Innern des Grundkörpers erzeugt, wobei die elastischen Rückstelleinrichtung die Kolbenstange entgegen der Auspressrichtung drückt, **dadurch gekennzeichnet, dass** die Vorspannung der Rückstelleinrichtung bei Betätigung der Kolbenstange (12) in Auspressrichtung durch einen in Auspressrichtung weisenden, mit der Kolbenstange (12) zusammenwirkenden, insbesondere von der Rückstelleinrichtung räumlich beabstandeten, Anschlag (18) begrenzt ist.

2. Applikationsvorrichtung nach Anspruch 1, **dadurch gekennzeichnet, dass** in der entspannten Stellung der Rückstelleinrichtung die Kolbenstange (12) an einem entgegen der Auspressrichtung weisenden Anlagebereich (41) der Rückstelleinrichtung anliegt und der Anschlag (18) des Kolbens (14) von einen entsprechenden Gegenanschlag (20) der Kolbenstange (12) entsprechend dem Weg der maximalen Vorspannung der Rückstelleinrichtung beabstandet ist.

3. Applikationsvorrichtung nach Anspruch 1, **dadurch gekennzeichnet, dass** insbesondere der Anlagebereich (41) des Rückstellelements (26) beim Aufbringen einer Auspresskraft von der Kolbenstange (12) in Auspressrichtung vorspannbar ist.

4. Applikationsvorrichtung nach einem der vorhergehenden Ansprüche, **dadurch gekennzeichnet, dass** die Rückstelleinrichtung in Anlagebereich (41) eine größere Wandstärke aufweist.

5. Applikationsvorrichtung nach einem der vorhergehenden Ansprüche, **dadurch gekennzeichnet, dass** die elastische Rückstelleinrichtung insbesondere mit einem verjüngten Außenumfang des Kolbens (14) fest verbunden ist.

6. Applikationsvorrichtung nach einem der vorhergehenden Ansprüche, **dadurch gekennzeichnet, dass** Kolben (14) und Kolbenstange (12) gewindefrei translatorisch zueinander und zu dem Grundkörper (28) gewindefrei axial verschieblich gelagert sind und die Treibkraft des Kolbens (14) gegenüber dem Grundkörper (28) größer als die Widerstandskraft des elastischen Rückstellelements (26) gegen Verformen ist.

7. Applikationsvorrichtung nach einem der vorhergehenden Ansprüche, **dadurch gekennzeichnet, dass** am Außenumfang des Kolbens (14) mindestens zwei Dichtrippen (31) umlaufend ausgebildet sind, die gegen den Grundkörper (28) wirken, und dass insbesondere an der Kolbenstange (12) und/oder an dem Kolben (14) eine umlaufende Aufnahmenut oder eine Aufnahmeschulter für die Aufnahme eines Dichtrings aus einem elastischen Material ausgebildet ist.

8. Applikationsvorrichtung nach einem der vorhergehenden Ansprüche, **dadurch gekennzeichnet, dass** die Kolbenstange (12) sich durch den Kolben (14) hindurch bis zum Anlagebereich (41) der Rückstelleinrichtung erstreckt und der Anschlag (18) zwischen Kolbenstange (12) und Kolben (14) im Verlauf des Kolbens (14), bevorzugt etwa in dessen axialer Mitte, gebildet ist.

9. Applikationsvorrichtung nach einem der vorhergehenden Ansprüche, **dadurch gekennzeichnet, dass** der Kolben (14) an seinem rückwärtigen Ende mindestens eine Gleitrippe aufweist, über welche er an der Kolbenstange (12) reibungsarm abgestützt ist.

10. Applikationsvorrichtung nach einem der vorhergehenden Ansprüche, **dadurch gekennzeichnet, dass** die Applikationsvorrichtung (10) eine Ausbringdüse (42) aufweist, die an einer in Auspressrichtung weisenden Seite des Grundkörpers (28) befestigt ist.

11. Applikationsvorrichtung nach einem der vorhergehenden Ansprüche, **dadurch gekennzeichnet, dass** der Grundkörper (28) bei abgenommener Ausbringdüse (42) befüllbar ist, und der Kolben (14) und die Kolbenstange (12) beim Befüllen zurückgedrückt wird.

12. Applikationsvorrichtung nach einem der vorhergehenden Ansprüche, **dadurch gekennzeichnet, dass** die Kolbenstange (12) wenigstens eine umlaufende Nut aufweist, die der Aufnahme eines sich an der Innenwandung des Grundkörpers (28) dichtend anliegenden Dichtungen dient.
